# EUROPEAN PATENT APPLICATION

(11) **EP 4 134 043 A1**
(43) Date of publication of application: **15.02.2023**
(21) Application number: 21784754.0
(22) Date of filing: 22.02.2021
(51) Int. Cl.: A61C 13/00, A61C 13/08, A61C 8/00

(54) **CUTTABLE ABUTMENT-INTEGRATED PROSTHETIC BLOCK AND METHOD FOR MANUFACTURING SAME**

(30) Priority: 10.04.2020 KR 20200043757
(71) Applicant: Hass Co., Ltd., Gangwon-do 25452 (KR)
(72) Inventor: KIM, Yong Su, Gangneung-si, Gangwon-do 25497 (KR); JEON, Hyun Jun, Busan 47508 (KR); LIM, Hyung Bong, Ansan-si, Gyeonggi-do 15521 (KR); KIM, Joon Hyung, Anseong-si, Gyeonggi-do 17560 (KR); KIM, Sung Min, Yongin-si, Gyeonggi-do 16903 (KR); SON, Si Won, Seoul 08846 (KR); KIM, Yena, Seoul 08250 (KR)
(74) Representative: Gulde & Partner
(86) International application number: PCT/KR2021/002178
(87) International publication number: WO 2021/206288

(57) **Abstract**

The present invention relates to a machinable abutment-integrated prosthetic block. The present invention provides a machinable abutment-integrated prosthetic block which is a machinable dental prosthetic block formed by integrating an abutment with a crown block made of a material requiring no heat treatment after machining, and is integrated through an adhesive layer containing a cured product formed of a polymerizable organic compound and thus can be processed by machining such as CAD/CAM processing in a state where the abutment is integrated with the crown. Therefore, the prosthetic block can be manufactured into artificial teeth such as temporary teeth or permanent teeth through machining in the block state while requiring no additional heat treatment after machining and can shorten a treatment time for implant restoration, thereby enabling a one-day prosthesis procedure.

## Description

### Technical Field

The present invention relates to a machinable abutment-integrated prosthetic block. The present invention provides a machinable abutment-integrated prosthetic block which is a machinable dental prosthetic block formed by integrating an abutment with a crown block made of a material requiring no heat treatment after machining and can be processed by machining such as CAD/CAM processing in a state where the abutment is integrated with the crown.

### Background Art

Improvement in aesthetics of the patient and time reduction for a one-day treatment are current issues in dentistry. To this end, materials and equipment regarding these issues have been developed proactively in dentistry. Therefore, materials such as zirconia, crystallized glasses, and composites, and equipment such as Computer-Aided Design/Computer-Aided Manufacturing (CAD/CAM), have been widely used. Such changes have facilitated improvements in aesthetic procedures requested by patients and time reduction in dental treatment due to one-day prosthesis treatment in dentistry. However, the situation is that additional methods from those currently used have been continuously requested by continuous demands from patients and what dentists need.

Generally, prostheses can be classified into inlays, onlays, veneers, crowns, etc. by the portion which each of the materials covers, and can be divided into temporary teeth and permanent teeth depending on purpose of use. Required physical properties vary according to each portion and purpose of use, and materials can be selected by physical properties. For example, composites and polymethyl methacrylate (PMMA) are advantageous since both composites and PMMA have excellent processability and aesthetics and can be implanted in an oral cavity without additional heat treatment after processing. However, both composite and PMMA have lower physical properties compared to other materials and are thus mainly used for inlays and onlays, which serve as temporary teeth. In the case of composites, research and development on composites with high physical properties have been continuously conducted. As a result, composites can be used for permanent teeth of crowns on premolar teeth. In the case of zirconia, even though zirconia is known as the material with the highest physical properties among dental prosthetic materials except for metal, it is mainly used as permanent teeth for posterior crowns due to its low aesthetics and the need for additional heat treatment after processing. However, to solve the above problems, the development of zirconia material that exhibits lower physical properties compared to the existing zirconia material but has improved aesthetics is in progress by changing the content of Y₂O₃ (yttria), etc. and commercialization thereof is in progress. Currently, zirconia and crystallized glass materials have one problem in common that a treatment time gets extended due to additional heat treatment after processing. As a result, solving the above problem is becoming another issue, and advanced enterprises are launching products that have solved this problem one by one.

Dental implantation is a method of fixing metal to the bone of gums and restoring an exposed area above the gums with a crown made of a dental prosthetic material. An implant is mainly composed of a crown, an abutment, and a fixture, and each part is connected by cementation and fixed screws. Crowns used in implantation are applied after placing implants with temporary and permanent teeth and before connecting the ultimately manufactured permanent teeth to prevent functional and aesthetic problems in advance. One of the problems in restoration when using such implants is that the treatment takes a long time.

On the other hand, the present applicant has endeavored to manufacture a crystallized glass block to which a superstructure is connected and filed a patent application for the crystallized glass block which is currently registered under Korean Patent No. 10-1796196. The patent document discloses a method of embedding a high-strength zirconia post that can serve as a core in a crystallized glass block to manufacture artificial teeth from the crystalized glass block through CAD/CAM processing and a method of coupling a metal link fastened to an implant fixture with the zirconia post. Specifically, in this case, the coupling to the zirconia post uses an inorganic binder and requires a heat treatment for 1 to 2 hours at a temperature in a range of 700°C to 900°C.

### Disclosure

### Technical Problem

The present invention is to provide a machinable abutment-integrated prosthetic block which can be processed by machining such as CAD/CAM processing in a state where the abutment is integrated with the crown. Therefore, the prosthetic block can be manufactured into artificial teeth such as temporary and permanent teeth through machining in a block state while requiring no heat treatment after machining and can shorten a treatment time for implant restoration, thereby enabling a one-day prosthesis procedure.

### Technical Solution

An embodiment of the present invention provides a machinable abutment-integrated prosthetic block which is a machinable dental prosthetic block formed by integrating an abutment with a crown block made of a material that requires no heat treatment after machining, wherein the integration is achieved through an adhesive layer comprising a cured product of a polymerizable organic compound. The cured product of a polymerizable organic compound comprises an organic matrix, which is a cured product of (a) two or more compounds selected from 2,2-bis[4-(2-hydroxy-3-methacryloyloxy propoxy) phenyl]propane (Bis-GMA), triethylene glycol dimethacrylate (TEGDMA), and diurethane dimethacrylate (UDMA); and (b)two or more compounds selected from 2-hydroxy ethyl methacrylate (HEMA), bis[2-[(2-methyl-1-oxoallyl)oxy]ethyl]dihydrogen benzene-1,2,4,5-tetracarboxylate (PMDM), and 10-methacryloyloxydecyl dihydrogen phosphate (10-MDP) and at least one inorganic filler selected from barium silicate (BaO₃Si) and silicon dioxide (SiO₂) dispersed in the organic matrix.

In a preferred embodiment of the present invention, each of the compounds (a) may be provided as a mixture of triethylene glycol dimethacrylate (TEGDMA) with at least one selected from 2,2-bis[4-(2-hydroxy-3-methacryloyloxy propoxy) phenyl] propane (bis-GMA) and urethanedimethacrylate (UDMA).

In a preferred embodiment of the present invention, the crown block made of a material that requires no heat treatment after machining, may include at least one material selected from polymethyl methacrylate-based polymer (PMMA), a ceramic-resin composite, zirconia, and crystallized glass.

In a preferred embodiment of the present invention, the ceramic-resin composite may be a cured product of at least two polymerizable organic compounds of selected from hydroxy ethyl methacrylate (HEMA), 2,2-bis[4-(2-hydroxy-3-methacryloyloxy propoxy)phenyl]propane (Bis-GMA), triethylene glycoldimethacrylate (TEGDMA), diurethanedimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters; and at least one inorganic material selected from barium silicate-based crystallized glass, leucite-based crystallized glass, alumina, zirconia, and glass.

In an embodiment of the present invention, the abutment may include at least one material selected from titanium, an alloy of titanium, and zirconia.

In a preferred embodiment of the present invention, the cured product of a polymerizable organic compound is a product produced by photocuring or thermal curing.

In a preferred embodiment of the present invention, a surface of the crown adjacent to the adhesive layer or a surface of the abutment adj acent to the adhesive layer may be a surface treated by acid etching or sandblasting.

The present invention also provides a method for manufacturing a machinable abutment-integrated prosthetic block which includes a step S1 of preparing a crown block made of a material that requires no heat treatment after machining, a step S2 of preparing an abutment, a step S3 of processing the crown block so that the crown has a hole conforming to the abutment, a step S4 of applying an adhesive composition containing a polymerizable organic compound onto the abutment, a step S5 of fastening the abutment coated with the adhesive composition to the hole-processed crown block, and a step S6 of integrating the abutment and the crown block by curing the crown block to which the abutment is fastened. The adhesive composition containing the polymerizable organic compound includes (a) two or more compounds selected from 2,2-bis[4-(2-hydroxy-3-methacryloyloxy propoxy) phenyl] propane (Bis-GMA), triethylene glycol dimethacrylate (TEGDMA), and diurethane dimethacrylate (UDMA), (b) two or more compounds selected from 2-hydroxy ethyl methacrylate (HEMA), bis[2-[(2-methyl-1-oxoallyl)oxy]ethyl]dihydrogen benzene-1,2,4,5-tetracarboxylate (PMDM), and 10-methacryloyloxydecyl dihydrogen phosphate (10-MDP), (c) at least one type of inorganic filler type selected from barium silicate (BaO₃Si) and silicon dioxide (SiO₂), and (d) a photoinitiator or a thermal initiator.

In the method for manufacturing a prosthetic block according to a preferred embodiment of the present invention, the hole-processed surface of the crown block in the step S3 may further be surface-treated by acid etching or sandblasting.

In the method for manufacturing a prosthetic block according to a preferred embodiment of the present invention, the surface of the abutment may be surface-treated by acid etching or sandblasting and may be coated with the adhesive composition containing the polymerizable organic compound in the step S4.

In an embodiment of the present invention, photocuring may be performed at a wavelength in a range of 350 nm to 450 nm, or thermal curing may be performed at a temperature in a range of 80°C to 150°C in the step S6.

The present invention also provides an abutment-integrated artificial tooth obtained by CAD/CAM processing or laser milling of the abutment-integrated prosthetic block according to an embodiment of the present invention.

In this case, the artificial tooth may be a temporary tooth or a permanent tooth.

### Advantageous Effects

A machinable abutment-integrated prosthetic block of the present invention can be processed by severe machining like machining such as CAD/CAM processing in a block state where the abutment is integrated with the crown. As a result, the machinable abutment-integrated prosthetic block can be manufactured into artificial teeth such as temporary teeth and permanent teeth suitable for individual patients while requiring no heat treatment after machining, and since implantation procedure can be finished by implanting an abutment-integrated crown after machining, a treatment time for implant restoration can be shortened, thereby enabling a one-day prosthesis procedure.

### Description of Drawings

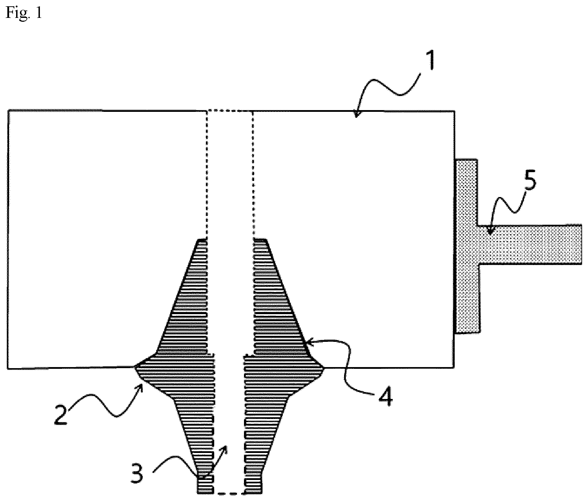

FIG. 1 is a schematic diagram illustrating a machinable abutment-integrated prosthetic block.

### Best Mode

Hereinafter, the present invention will be described in more detail with reference to the drawing.

The foregoing and further aspects of the present invention will become more apparent through the preferred embodiments described with reference to the accompanying drawing. Hereinafter, the embodiment of the present invention will be described in detail so that those skilled in the art can easily understand and reproduce the present invention.

A structure of a typically operated implant is divided into a fixture, an abutment, and a crown. The fixture is embedded in the alveolar bone and is osseointegrated over time, and serves as a support. The abutment, the part to which the top of the fixture is connected, serves to connect the fixture to the crown which serves as a tooth, and is generally connected to the crown through cementation. Such procedures are generally performed in stages over a long period of time.

FIG. 1 is a schematic diagram illustrating a machinable abutment-integrated prosthetic block according to the present invention, which is a machinable dental prosthetic block formed by integrating an abutment 2 and a crown block 1 made of a material that requires no heat treatment after machining. The integration is achieved through an adhesive layer 4 which contains a cured product of a polymerizable organic compound.

It is obvious that such a prosthetic block may include a screw access channel 3 ultimately in consideration of connection of the fixture, and be prepared with a mandrel 5 in consideration of machining.

To perform machining such as CAD/CAM processing or laser milling in a state where the abutment 2 made of various materials is integrated with the crown block 1 made of a material that requires no heat treatment after machining, each of the integration methods and materials may play significant roles.

In this aspect, the adhesive layer preferably includes the cured product of the polymerizable organic compound. That is, the abutment 2 and the crown block 1 are preferably integrated through a curing reaction of the polymerizable organic compound. Particularly, in this case, depending on a composition of the cured product, the abutment 2 and the crown block 1 may overcome the material difference and be bonded to each other. Specifically, the cured product preferably includes an organic matrix, which is a cured product of (a) two or more compounds selected from 2,2-bis[4-(2-hydroxy-3-methacryloyloxy propoxy) phenyl]propane (Bis-GMA), triethylene glycol dimethacrylate (TEGDMA), and diurethane dimethacrylate (UDMA); and (b)two or more compounds selected from 2-hydroxy ethyl methacrylate (HEMA), bis[2-[(2-methyl-1-oxoallyl)oxy]ethyl]dihydrogen benzene-1,2,4,5-tetracarboxylate (PMDM), and 10-methacryloyloxydecyl dihydrogen phosphate (10-MDP) and at least one inorganic filler selected from barium silicate (BaO₃Si) and silicon dioxide (SiO₂) dispersed in the organic matrix.

In this case, preferably the compounds (a) are organic compounds allowing each material to exhibit a viscosity capable of being applied and each of the compound (a) is provided as a mixture of compounds having low viscosity and compounds having high viscosity. Specifically, each of the compounds (a) is provided as a mixture of triethylene glycol dimethacrylate (TEGDMA) and at least one selected from 2,2-bis[4-(2-hydroxy-3-methacryloyloxy propoxy) phenyl]propane (Bis-GMA) and urethanedimethacrylate (UDMA).

The compound (b) may act as an adhesive compound that improves adhesion of each material.

Also, it is preferable to include at least one type of inorganic filler selected from barium silicate (BaO₃Si) and silicon dioxide (SiO₂) to improve the physical properties of the adhesive layer from a perspective of improving the adhesive strength between organic and inorganic materials.

In the case of the adhesive layer 4, it is obvious that each content of the organic matrix and the inorganic filler, and the mixing ratio of the compounds (a) in a mixture form and the compounds (b) constituting the organic matrix may be appropriately adjusted in consideration of each material of the crown block and the abutment.

In such an adhesive layer, the cured product of a polymerizable organic compound may be a product produced by photocuring or thermal curing.

According to the machinable abutment-integrated prosthetic block of the present invention, the crown block 1 is preferably made of a material that requires no heat treatment after machining. It is because when the material only exhibits required properties only after undergoing additional heat treatment in a block state where the crown has a shape conforming to the abutment through machining, it may contain a problem that the size of the crown block after additional heat treatment can be changed by circumstances. The crown block of the prosthetic block, according to one aspect of the present invention, preferably includes at least one material selected from polymethyl methacrylate-based polymer (PMMA), a ceramic-resin composite, zirconia, and crystallized glass. Particularly, in the case of the crystallized glass, it is preferable that the crystallized glass is capable of being cut while containing lithium disilicate crystal as the main crystal.

In this case, the PMMA polymer has lower physical properties than other materials, so it is generally applicable as a block for temporary teeth. Temporary teeth implanted in the oral cavity after implantation and before using permanent teeth prostheses are used to check whether the fixture and the periodontal portion are well fixed and if there is any pain in the patient during mastication. Temporary teeth are also used for aesthetic functions before manufacturing permanent teeth prostheses. Each of the ceramic-resin composite, zirconia, and crystallized glass is a material that exhibits higher physical properties than the PMMA polymer and can constitute the crown block 1 which can be applied as a permanent tooth prosthesis.

More specifically, the ceramic-resin composite may be a cured product of at least two or more polymerizable organic compounds selected from hydroxy ethyl methacrylate (HEMA), 2,2-bis[4-(2-hydroxy-3-methacryloyloxy propoxy) phenyl] propane (Bis-GMA), triethylene glycoldimethacrylate (TEGDMA), diurethanedimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters; and at least one inorganic material selected from barium silicate-based crystallized glass, leucite-based crystallized glass, alumina, zirconia, and glass. Such a ceramic-resin composite is a material applicable to anterior teeth and premolar teeth. It is obvious that the contents described in Korean Patent Application Publication No. 10-2017-0173152 by the present applicant may be referred to in detail.

In the case of zirconia as the material of the crown block 1, it is preferable to contain 2 mol% to 10 mol% of yttria, which can be applied immediately after machining without additional heat treatment so that it can be used in the posterior region which generally requires high physical properties.

On the other hand, the material of the abutment 2 of the machinable abutment-integrated prosthetic block according to the present invention is not limited and may include at least one material selected from titanium, an alloy of titanium, and zirconia as one example.

When the abutment 2 and the crown block 1 of the above materials are integrated through the adhesive layer 4 containing the cured product of the polymerizable organic compound, a surface of the crown adjacent to the adhesive layer 4 or a surface of the abutment adjacent to the adhesive layer 4 is preferably a surface treated by acid etching or sandblasting for the purpose of improving the interlayer adhesion.

A method for manufacturing such a machinable abutment-integrated prosthetic block includes a step S1 of preparing a crown block made of a material requires no heat treatment after machining, a step S2 of preparing an abutment, a step S3 of processing the crown block so that the crown block has a hole having a shape conforming to the abutment, a step S4 of applying an adhesive composition containing a polymerizable organic compound onto the abutment, a step S5 of fastening the abutment coated with the adhesive composition to the hole-processed crown block, and a step S6 of integrating the abutment and the crown block by curing the crown block to which the abutment is fastened.

In this case, the adhesive composition containing the polymerizable organic compound preferably includes (a) two or more compounds selected from 2,2-bis[4-(2-hydroxy-3-methacryloyloxy propoxy) phenyl] propane (Bis-GMA), triethylene glycol dimethacrylate (TEGDMA), and diurethane dimethacrylate (UDMA), (b) two or compounds more selected from 2-hydroxy ethyl methacrylate(HEMA), bis[2-[(2-methyl-1-oxoallyl)oxy]ethyl]dihydrogen benzene-1,2,4,5-tetracarboxylate (PMDM), and 10-methacryloyloxydecyl dihydrogen phosphate (10-MDP), (c) at least one type of inorganic filler selected from barium silicate (BaO₃Si) and silicon dioxide (SiO₂), and (d) a photoinitiator or a thermal initiator.

In this case, the compounds (a), (b), and (c) are as described above.

When (d) is a photoinitiator, it is preferably a compound or polymerization initiating system in which curing is initiated at a wavelength in a range of 350 nm to 450 nm. Camphorquinone (CQ) can be one example of the photoinitiator. The polymerization initiating system may include at least one or more photosensitizer selected from 1-phenyl-1,2-propanedione (PPD), diphenyliodonium hexafluorophosphate (DPIHP), and p-octyloxy-phenyl-phenyl iodonium hexafluoroantimonate (OPPI), and may include a catalyst which is tertiary amine such as ethyl-4-dimethylaminobenzoate (EDMAB), which is a relatively hydrophobic aromatic compound, or 2-(dimethylamino)ethyl methacrylate (DMAEMA), which is a hydrophilic aliphatic compound.

On the other hand, thermal curing is preferably performed with the use of the thermal initiator at a temperature in a range of 80°C to 150°C and may include a peroxide-based initiator such as benzoyl peroxide (BPO) as an example.

In the step S1 of preparing the crown block of the material that requires no heat treatment after machining, the material related to the crown block 1 is as described above. In this case, the crown block may have the mandrel 5 attached thereto.

In addition, in the step S2 of preparing the abutment, the abutment 2 and the related materials are as described above.

In the step S3 of processing the prepared crown block so that the crown has a hole having a shape conforming the abutment, the hole-processing is performed conforming the structure so that the abutment is fixed to the crown block 1 which is typically manufactured with the mandrel 5 attached thereto. In this case, in the expression "conforming the abutment", the abutment may be understood as a structure of the abutment including the screw access channel 3 to be connected to the fixture in consideration of the ultimate implantation of the prosthesis.

As described above, the surface of the crown block adj acent to the adhesive layer 4 or a surface of the abutment adjacent to the adhesive layer 4 is preferably a surface-treated layer. In this regard, it is preferable to perform surface treatment of the hole-processed surface of the crown block by acid etching or sandblasting and fasten the surface of the crown block to the abutment 2 coated with the adhesive composition.

On the other hand, in the step S4 of applying the adhesive composition containing the polymerizable organic compound onto the abutment, it is preferable to perform surface treatment of the abutment by acid etching or sandblasting and apply the adhesive composition containing the polymerizable organic compound onto the surface of the abutment, in the same context.

In this case, the surface treatment is preferably performed in both steps S3 and S4 or may be performed only in one of the steps.

After the step (S5) of fastening the abutment coated with the adhesive composition to the hole-processed crown block, the step S6 includes integration of the abutment and the crown block by curing the crown block to which the abutment is fastened

As the simultaneous curing reaction integrates the abutment and the crown block, the interlayer bonding strength may be effectively enhanced.

In the step S6, it is preferable that photocuring is performed at a wavelength in a range of 350 nm to 450 nm, or thermal curing is performed at a temperature in a range of 80°C to 150°C

According to the manufacturing method as described above, the integration of the crown block 1 made of various materials and the abutment 2 made of various materials by a curing reaction of a specific polymerizable organic compound is advantageous in that, as a relatively mild process enables the integration without severe heat treatment, concerns about changes in the physical properties of the crown block material can be dispelled.

In addition, the machinable abutment-integrated prosthetic block manufactured by the same method as above can be directly processed by machining such as CAD/CAM processing or laser milling depending on the needs of the patient to produce an abutment-integrated artificial tooth. When the abutment-integrated prosthetic block is connected to the fixture, a treatment time for implant restoration can be shortened, thereby enabling a one-day implant procedure.

Particularly, even when machining such as CAD/CAM processing or laser milling is involved, the prosthetic block in the abutment-integrated state can maintain mechanical stability by having excellent adhesive strength and shear bonding strength. Also, the integrated body of the abutment and the crown can reduce the possibility of secondary infection of bacterial infiltration resulting from individual procedures.

It is obvious that the artificial tooth obtained by machining the abutment-integrated prosthetic block may be a temporary tooth or a permanent tooth depending on the crown block material.

Although the present invention has been described with reference to one embodiment shown in the drawing, this is merely exemplary, and those skilled in the art will understand that various modifications and equivalent other embodiments are possible therefrom.

### Industrial Applicability

The present invention relates to a machinable abutment-integrated prosthetic block. Provided is a machinable abutment-integrated prosthetic block which is a machinable dental prosthetic block formed by integrating an abutment and a crown block made of a material that requires no heat treatment after machining, and can be processed by machining such as CAD/CAM processing in a state where the abutment is integrated with the crown.

The machinable abutment-integrated prosthetic block of the present invention can be processed by severe machining like machining such as CAD/CAM processing in a block state where the abutment is integrated with the crown. Therefore, the machinable abutment-integrated prosthetic block can be manufactured into artificial teeth such as temporary and permanent teeth suitable for individual patients while requiring no additional heat treatment after machining, and since implantation procedure can be finished by implanting an abutment-integrated crown after machining, a treatment time for implant restoration can be shortened, thereby enabling a one-day prosthesis procedure.

## Claims

1. A machinable abutment-integrated prosthetic block which is a machinable dental prosthetic block formed by integrating an abutment and a crown block made of a material requiring no heat treatment after machining,
wherein the integration is achieved through an adhesive layer comprising a cured product of a polymerizable organic compound,
wherein the cured product of a polymerizable organic compound comprises:
an organic matrix, which is a cured product of (a) two or more compounds selected from 2,2-bis[4-(2-hydroxy-3-methacryloyloxy propoxy) phenyl]propane (Bis-GMA), triethylene glycol dimethacrylate (TEGDMA), and diurethane dimethacrylate (UDMA); and (b)two or more compounds selected from 2-hydroxy ethyl methacrylate (HEMA), bis[2-[(2-methyl-1-oxoallyl)oxy]ethyl]dihydrogen benzene-1,2,4,5-tetracarboxylate (PMDM), and 10-methacryloyloxydecyl dihydrogen phosphate (10-MDP) and
at least one inorganic filler selected from barium silicate (BaO₃Si) and silicon dioxide (SiO₂) dispersed in the organic matrix.

2. The prosthetic block of claim 1, wherein each of the compounds (a) is provided as a mixture of triethylene glycol dimethacrylate (TEGDMA) with at least one selected from 2,2-bis[4-(2-hydroxy-3-methacryloyloxy propoxy) phenyl] propane (bis-GMA) and urethanedimethacrylate (UDMA).

3. The prosthetic block of claim 1, wherein the crown block made of a material that requires no heat treatment after machining comprises at least one material selected from polymethyl methacrylate-based polymer (PMMA), a ceramic-resin composite, zirconia, and crystallized glass.

4. The prosthetic block of claim 3, wherein the ceramic-resin composite is a cured product of
at least two polymerizable organic compounds selected from hydroxy ethyl methacrylate (HEMA), 2,2-bis[4-(2-hydroxy-3-methacryloyloxy propoxy)phenyl]propane (Bis-GMA), triethylene glycoldimethacrylate (TEGDMA), diurethanedimethacrylate (UDMA), urethane dimethacrylate (UDM), biphenyldimethacrylate (BPDM), n-tolylglycine-glycidylmethacrylate (NTGE), polyethylene glycol dimethacrylate (PEG-DMA), and oligocarbonate dimethacrylic esters; and
at least one inorganic material selected from barium silicate-based crystallized glass, leucite-based crystallized glass, alumina, zirconia, and glass.

5. The prosthetic block of claim 1, wherein the abutment comprises at least one material selected from titanium, alloys of titanium, and zirconia.

6. The prosthetic block of claim 1, wherein the cured product of a polymerizable organic compound is a product produced by photocuring or thermal curing.

7. The prosthetic block of claim 1, wherein a surface of the crown adj acent to the adhesive layer or a surface of the abutment adjacent to the adhesive layer is a surface treated by acid etching or sandblasting.

8. A method for manufacturing a machinable abutment-integrated prosthetic block, the method comprising:
a step S 1 of preparing a crown block made of a material that requires no heat treatment after machining;
a step S2 of preparing an abutment;
a step S3 of processing the crown block so that the crown has a hole having a shape conforming to the abutment;
a step S4 of applying an adhesive composition comprising a polymerizable organic compound onto the abutment,
a step S5 of fastening the abutment coated with the adhesive composition to the hole-processed crown block; and
a step S6 of integrating the abutment and the crown block by curing the crown block to which the abutment is fastened,
wherein the adhesive composition comprising the polymerizable organic compound comprises (a) two or more compounds selected from 2,2-bis[4-(2-hydroxy-3-methacryloyloxy propoxy) phenyl] propane (Bis-GMA), triethylene glycol dimethacrylate (TEGDMA), and diurethane dimethacrylate (UDMA), (b) two or more compounds selected from 2-hydroxy ethyl methacrylate(HEMA), bis[2-[(2-methyl-1-oxoallyl)oxy]ethyl]dihydrogen benzene-1,2,4,5-tetracarboxylate (PMDM), and 10-methacryloyloxydecyl dihydrogen phosphate (10-MDP), (c) at least one inorganic filler selected from barium silicate (BaO₃Si) and silicon dioxide (SiO₂), and (d) a photoinitiator or a thermal initiator.

9. The method of claim 8, wherein in the step S3, the hole-processed surface of the crown block is further surface-treated by acid etching or sandblasting.

10. The method of claim 8, wherein in step S4, the surface of the abutment is surface-treated by acid etching or sandblasting and then coated with the adhesive composition comprising the polymerizable organic compound.

11. The method of claim 8, wherein in the step S6, photocuring is performed at a wavelength in a range of 350 nm to 450 nm.

12. The method of claim 8, wherein in the step S6, thermal curing is performed at a temperature in a range of 80°C to 150°C.

13. An abutment-integrated artificial tooth obtained by CAD/CAM processing or laser milling of the machinable abutment-integrated prosthetic block of claim 1.

14. An abutment-integrated artificial tooth obtained by CAD/CAM processing or laser milling of the machinable abutment-integrated prosthetic block manufactured by the method of claim 8.

15. The abutment-integrated artificial tooth of any one of claims 13 to 14, wherein the artificial tooth is a temporary tooth or a permanent tooth.
